# FASCICULE DE BREVET EUROPEEN

(11) **EP 3 097 179 B1**
(45) Date de publication et mention de la délivrance du brevet: **18.10.2017**
(21) Numéro de dépôt: 15700762.6
(22) Date de dépôt: 22.01.2015
(51) Int. Cl.: C12M 1/12, C12M 1/00, C12Q 1/04, G01N 33/52

(54) **DISPOSITIF POUR LA CULTURE DE MICROORGANISMES ET PROCEDE ASSOCIE**
VORRICHTUNG ZUR KULTIVIERUNG VON MIKROORGANISMEN UND ZUGEHÖRIGES VERFAHREN
DEVICE FOR CULTURING MICROORGANISMS AND ASSOCIATED METHOD

(30) Priorité: 20.01.2014 FR 1450422
(43) Date de publication de la demande: 30.11.2016
(73) Titulaire: Biomérieux, 69280 Marcy l'Étoile (FR); Arjo Wiggins Fine Papers Limited, Manchester M1 5ES (GB)
(72) Inventeur: MONTET, Marie-Pierre, F-69200 Venissieux (FR); ROZAND, Christine, F-69290 St Genis les Ollières (FR); DEPRES, Gael, F-38850 Chirens (FR); VAU, Jean-Marie, F-75012 Paris (FR); BAUMLIN, Jean-Marie, F-62223 Anzin Saint Aubin (FR)
(86) Numéro de dépôt international: PCT/EP2015/051295
(87) Numéro de publication internationale: WO 2015/107228

(56) Documents cités:
- WO-A1-96/29427
- FR-A1- 2 766 204
- US-A1- 2002 192 742

## Description

La présente invention concerne, de façon générale, le domaine du diagnostic in vitro et plus précisément celui du diagnostic microbiologique. Plus particulièrement, elle porte sur un dispositif pour la culture de microorganismes issus d'un échantillon contaminé.

Dans les domaines du diagnostic clinique et du contrôle microbiologique industriel agroalimentaire, pharmaceutique ou cosmétique, les milieux de culture gélifiés en boite de Petri, le plus souvent gélosés, constituent depuis la fin du XIXème siècle un outil indispensable à la détection et à l'identification des micro-organismes pathogènes.
Les milieux gélosés pour la croissance de micro-organismes sont largement répandus. Néanmoins leur préparation est chronophage car ils doivent être préparés juste avant leur utilisation afin d'assurer leur stérilité.
Il existe également des géloses pré-préparées. Ces dernières coutent chères et ont une durée de péremption courte.
Plusieurs produits ont été proposés commercialement pour remplacer un milieu de culture en boite de Petri. Ainsi, la société 3M propose le système Petrifilm^{™} composé de deux parties, un film inférieur et un film supérieur recouverts en surface par certains composants du milieu de culture déshydraté. Au moment de l'analyse, l'échantillon est déposé entre ces deux films.
Un autre système développé par la société Nissui Pharmaceutical, le Compact Dry^{™}, consiste également en un milieu déshydraté.
Ces milieux de culture ont pour avantage de se conserver plus longtemps qu'un milieu de culture gélosé prêt à l'emploi. Ils peuvent également présenter un faible encombrement et ainsi utiliser un faible espace d'incubation.
Néanmoins, ces milieux de culture ne permettent pas d'effectuer un isolement de microorganismes par frottement d'un moyen mécanique sur le milieu.
En effet, l'isolement de microorganismes sur milieu de culture gélifié, à partir d'un échantillon à analyser ou d'une suspension de microorganismes, est une étape souvent indispensable à de nombreux procédés d'analyse microbiologique. Cette étape est notamment utilisée pour réaliser des identifications, vérifier la pureté microbienne d'un échantillon ou encore effectuer un dénombrement bactérien par comptage des colonies isolées ainsi obtenues.
Les techniques d'isolement ont pour objectif d'obtenir à la surface d'un milieu nutritif gélifié des colonies directement utilisables (CDU) pour identifier et déterminer la sensibilité aux antibiotiques. Elles sont bien connues de l'homme du métier, la technique des stries (ou cadrans) étant la technique de référence. Cette dernière consiste à déposer l'inoculum par frottement sur une surface avec une probabilité égale par unité de surface parcourue. La densité locale distribuée diminue approximativement de façon exponentielle lors du parcours de l'instrument. Ainsi, plusieurs zones d'étalement à partir d'un seul inoculum sont réalisées, avec ou sans chevauchements des zones, afin d'obtenir l'effet adéquat de distribution et un appauvrissement en bactéries lors du segment d'étalement subséquent. En fin d'étalement les cellules sont suffisamment isolées les unes des autres pour que les développements microbiens sous forme de CDU (colonies visibles ou microcolonies) ne soient pas superposées, même partiellement. Cette technique peut être aussi réalisée par un seul ensemencement continu en spirale au moyen d'une platine tournante ou par une bille magnétique entraînée par un dispositif produisant un ensemencement continu non recouvrant ou éventuellement partiellement chevauchant.
Une autre technique largement répandue consiste en l'isolement sur boîte de milieu gélifié par étalement en surface. Dans ce cas, un mélange de cellules à une concentration cellulaire faible permettant de mettre en culture 30 à 300 cellules, est étalé à la surface du gel d'une boîte de Petri de 9 cm de diamètre, chaque cellule se développant en une colonie isolée. Lorsque moins de 30 cellules sont mises en contact avec le gel nutritif, des problèmes statistiques faussent l'exactitude du dénombrement. Lorsque l'effectif dénombré est au-delà de 300 cellules, des erreurs de dénombrement apparaissent du fait du chevauchement des surfaces des colonies. L'étalement est habituellement réalisé par un instrument comportant par exemple une partie linéaire qui est en contact avec le gel ou par emploi de billes de quelques millimètres de diamètre roulant aléatoirement sur la surface par un mouvement désordonné. Cette technique n'est adaptée qu'à un échantillon faiblement contaminé ou dilué car un nombre élevé de cellules augmente la probabilité de confluence des colonies résultant de la croissance.

Il est également possible d'effectuer un isolement sur boite par inoculation en profondeur. L'échantillon de départ est dilué plusieurs fois de manière à réduire suffisamment la population microbienne et obtenir des colonies séparées. De petit volumes de chacun des échantillons dilués sont alors mélangés à un gel liquide, habituellement de la gélose maintenue en surfusion environ à 45°C. Les mélanges sont immédiatement versés dans des boites de culture stériles et après gélification et incubation, chaque cellule est immobilisée et forme une colonie.
Certaines méthodes manuelles se sont vues automatisées grâce à la mise au point de dispositif. C'est l'objet, par exemple, du document EP-0 242 114 qui décrit un appareil et une méthode d'ensemencement d'un milieu de culture avec un échantillon. La méthode consiste à réaliser plusieurs segments d'étalement à partir d'un inoculum. Ces segments sont sous la forme d'arc de cercle et sont réalisés au moyen de quatre têtes d'étalement différentes. Un effet de dilution de l'échantillon est obtenu par chevauchement partiel des segments subséquents. La méthode décrite dans le document est en fait très proche de la méthode d'isolement manuelle de référence.
Plus récemment, de nouvelles méthodes d'isolement ont vu le jour, permettant l'amélioration de l'épuisement bactérien par l'usage d'un applicateur optimisé tel que décrit dans le document WO-A-2005071055. C'est le cas notamment de la méthode d'ensemencement mise en oeuvre dans l'automate commercialisé par la demanderesse bioMérieux sous la référence PREVI^{™} Isola.
Néanmoins, ces techniques d'isolement ne sont efficaces que sur des milieux de culture gélifiés.
En effet, l'isolement sur des systèmes non gélosés tels que le Petrifilm^{™} présentent plusieurs inconvénients. L'isolement de colonies sur ces milieux n'est possible que par inclusion dans le gel formé lors de la réhydratation, et donc à partir d'un échantillon de départ faiblement contaminé ou ayant subi une série de dilutions. La concentration finale, à déposer sur le milieu, doit être inférieure à 300 cfu/ml (Unité Formant Colonie), ces données classiques dépendant de la taille de la colonie.
De plus, ces milieux ne peuvent subir la contrainte mécanique d'un moyen d'isolement par épuisement sans être détériorés. Ainsi, une des problématiques majeures de ces milieux de culture réhydratables est qu'ils ne sont pas compatibles avec l'isolement mécanique manuel ou automatisé de micro-organismes. Ils nécessitent, lorsque l'échantillon de départ est largement contaminé (au-delà de 300 Unités Formant Colonie/ml), la réalisation d'une série de dilutions, impliquant une prise d'essai plus importante, une perte de temps et la consommation d'un nombre important de réactifs (milieu de culture, tubes de diluants, etc.), générateur d'un volume élevé de déchets (autoclavage, coût du traitement). De plus, si un grand nombre de dilutions est réalisé, il existe un risque de perdre, par l'effet de la dilution le microorganisme pathogène cible, si celui-ci est présent en faible quantité par rapport à la micro flore totale.
De la même manière, il est connu dans l'art antérieur l'ajout d'une membrane filtrante sur un milieu gélosé. Ainsi, pour le dénombrement de bactéries dans de l'eau, une membrane filtrante est utilisée afin de collecter les microorganismes à sa surface. Cette membrane est ensuite placée sur une gélose. La membrane filtrante permet au nutriment de la gélose de la traverser afin de faire croitre les microorganismes sous forme de colonies sur sa surface.
Alternativement, la membrane peut être placée sur un matériau fibreux absorbant contenant du nutriment déshydraté qui est humidifié avec une quantité déterminée d'eau.
Ces membranes sont réalisés par des techniques de perforation de film plastique tel que par laser ou faisceaux d'électrons, ou en assemblant des fibres pour réaliser la fonction de filtration.
On peut citer par exemple les micros filtres Sartorius constitués d'un plastique composé d'un réseau polymérique présentant différentes tailles de pore. Ces systèmes présentent notamment l'inconvénient d'instabilité, la membrane déposée sur le milieu de culture pouvant présenter des déformations et des plissements. Le transfert et la manipulation de la membrane ne sont pas aisés et sont un risque de contamination. De plus ces systèmes ne permettent pas non plus l'isolement par contact avec un moyen d'isolement du fait de la fragilité de la membrane.
Le document FR2182073, propose un dispositif comprenant une membrane micro-poreuse associée par une zone interfaciale continue à une couche absorbante.

Le document US2002/192742 divulgue un dispositif comprenant un support et un milieu nutritif, ledit support comprenant un substrat fibreux hydrophile et au moins une couche poreuse en contact avec l'une des faces du substrat fibreux et comprenant un pigment ou un mélange de pigments. Cette structure a trois strates, ce qui a pour inconvénient d'éloigner les nutriments de la zone effective de croissance.

Eu égard à la somme des problématiques développées supra, la présente invention propose un nouveau dispositif de culture de micro-organismes.

Ainsi, un objectif de la présente invention est de fournir un dispositif et un procédé robustes permettant la croissance de microorganismes.

Un objectif de l'invention est de permettre également l'isolement de microorganismes sur un milieu de culture réhydratable, ou réhydraté peu avant ou simultanément à l'isolement microbien.

Un autre objectif de la présente invention est de fournir un procédé d'isolement de microorganismes à partir d'un échantillon ayant une concentration microbienne initiale élevée.

### Exposé de l'invention

Ces objectifs parmi d'autres sont atteints par la présente invention qui propose un dispositif pour la culture et/ou l'isolement et/ou la détection et/ou l'identification et/ou le dénombrement d'au moins un microorganisme cible dans un échantillon susceptible de le contenir caractérisé en ce qu'il comprend un support et un milieu nutritif ;
ledit support comprenant :
- un substrat fibreux hydrophile,
- au moins une couche poreuse en contact avec l'une des faces du substrat fibreux, comprenant un pigment ou un mélange de pigments, et au moins un liant, ledit pigment ayant une taille inférieure à 5 µm et la quantité dudit pigment ou dudit mélange de pigments étant comprise entre 50 et 97% en poids sec par rapport au poids sec de la couche poreuse ;
ledit milieu nutritif étant compris dans le substrat fibreux.

On entend par substrat fibreux des fibres de petites tailles jointives plus ou moins entrelacées constituant un ensemble ayant une intégrité mécanique et pouvant être traversé par un liquide. Préférentiellement le substrat fibreux comprend des fibres de cellulose, notamment de coton.

Le substrat fibreux doit être hydrophile afin qu'il puisse absorber une solution aqueuse d'un milieu nutritif et alimenter uniformément sa surface. Le substrat fibreux peut servir de réservoir de milieu nutritif. Le substrat fibreux doit être suffisamment hydrophile et absorbant pour que la surface du système s'humidifie rapidement lorsque le substrat fibreux est mis en contact avec un milieu liquide.
Avantageusement, le milieu nutritif compris dans le substrat fibreux est déshydraté. Ainsi le dispositif selon l'invention présente l'avantage d'avoir une plus grande durée de vie que la gélose pré préparée. Il peut également être irradié afin d'être stérilisé.

Le rôle de la couche poreuse est de retenir les microorganismes à sa surface tout en permettant à l'eau chargée de nutriment compris dans le substrat fibreux de nourrir ces microorganismes. Ainsi, la couche poreuse comprend un pigment ou un mélange de pigments, ledit pigment ayant une taille inférieure à 5 µm et la quantité dudit pigment ou du mélange de pigments étant comprise entre 50 et 97% en poids sec par rapport au poids sec de la couche poreuse.
Les pigments, par leur nature, leur taille, leur arrangement stérique dans la couche poreuse vont alors empêcher les microorganismes de migrer de la surface vers le substrat fibreux tout en étant perméable aux éléments compris dans le substrat fibreux situé sous cette couche.
Préférentiellement, la couche a des pores inferieures à 600 nm, préférentiellement inférieure à 400nm. Elle a également suffisamment de tortuosités pour que les microorganismes ne puissent pas la traverser.
Le choix des pigments et la quantité de ceux-ci à la surface du substrat fibreux évitent également que le liquide soit libre à la surface de la couche poreuse ce qui entrainerait la croissance de colonies en étoile.
Préférentiellement, la couche poreuse est solidaire avec l'une des faces du substrat fibreux. Ainsi, la couche poreuse et le substrat forme un tout et ne peuvent pas être séparés l'un de l'autre sans détériorer l'un et/ou l'autre.

On entend par « pigments » des composés solides, normalement insolubles dans l'eau (à l'exception du CaCO₃ qui est un pigment soluble dans l'eau acide), de petites tailles typiquement comprises entre 0,1 et 5µm, dont la taille, la forme, la distribution de taille, dépendent de la nature chimique, la provenance et le mode de fabrication. Usuellement on distingue les pigments organiques des pigments inorganiques.
Les pigments organiques sont des pigments plastiques sous forme de billes pleines ou creuses.
A titre d'exemple les pigments inorganiques peuvent être choisis parmi les pigments suivants : carbonates de calcium broyés, carbonate de calcium précipités, kaolin, silice, talc, oxyde de zinc, sulfate de baryum, dioxyde de titane.
Préférentiellement, les pigments sont des pigments inorganiques choisis parmi les pigments suivants : kaolin, talc, dioxyde de titane, carbonate de calcium.
La quantité dudit pigment ou du mélange de pigments est comprise entre 50 et 97% en poids sec par rapport au poids sec de la couche poreuse, préférentiellement entre 60 et 95%, encore préférentiellement entre 80 et 90%.
Au moins un des pigments a une taille inférieure à 5 micromètres, préférentiellement inférieure à 3 micromètres, encore plus préférentiellement inférieure à 2 micromètres. Lors de leurs dépôts, les pigments se mettent les uns sur les autres laissant place à des pores ouverts entre eux. La taille des pores dépendant principalement de la forme des pigments, de leur taille moyenne et de leur distribution de taille.
Ainsi, la forme des pigments affecte le volume des pores. En théorie, des pigments parfaitement cubiques et de même dimension pourraient s'assembler sans laisser d'espace entre eux. Dans le cas de sphères de même taille, si elles sont assemblées au plus prêt les unes des autres, 26% de l'espace est occupé par les interstices. De même si les particules solides sont constituées de pigments gros et petits, les petits pigments pourraient se loger entre les gros pigments comblant ainsi le volume des interstices et réduisant le volume des pores. La taille des interstices entre les pigments est typiquement plus petite que les pigments eux même. Ainsi, en utilisant des pigments fins, on crée des pores de plus petites tailles qu'en utilisant des pigments grossiers. On peut alors, en fonction du type de microorganisme ciblé, choisir une porosité moyenne de la couche plus ou moins élevée, comme par exemple une porosité moyenne supérieure au micron pour la croissance de levure.
D'autre part, les pigments peuvent se joindre les uns aux autres de manière plus ou moins compacte. De la même manière que l'on secoue une boite contenant des grains pour les compacter, le séchage plus ou moins rapide de la couche peut influencer l'agencement des pigments les uns par rapport aux autres dans leur phase de consolidation et modifier le volume poreux.

Les pigments sont liés entre eux à l'aide d'un liant comme par exemple du latex styrène butadiène, du latex styrène acrylique, du carboxyle méthyle cellulose, de l'alcool polyvinylique, de l'amidon ou de la gélatine. Préférentiellement le liant est du latex styrène butadiène et/ou du latex styrène acrylique et/ou du carboxyle méthyle cellulose. La quantité de liant peut également moduler la porosité de la couche. Ainsi, si une couche est constituée de pigment et saturée en liant, le liant comble les interstices entre les pigments solides, et peut constituer alors un ensemble compact plein et solide une fois sec. Lorsque la quantité de liant est faible par rapport au pigment, il se crée des creux entre les pigments que le liant en faible quantité, ne peut pas entièrement combler. L'ensemble de ces creux constitue une porosité interstitielle qui peut être caractérisée entre autre, par la dimension de ces pores et par l'ensemble du volume des pores. Ainsi, lorsque la quantité de liant est faible par rapport au pigment de la couche, elle assure une certaine porosité interstitielle entre les pigments.
De préférence, la quantité de liant est comprise entre 3 et 25% en poids sec par rapport au poids sec de la couche poreuse, préférentiellement entre 5 et 15%. Préférentiellement, la quantité de pigments et/ou de liants est comprise entre 30g/m² et 90 g/m², préférentiellement entre 50g/m² et 80 g/m².

D'autres éléments peuvent être ajoutés à la couche poreuse tels qu'un réticulant, un épaississant, un antimousse, un tensioactif. Ces éléments sont adaptés au processus de couchage.
De façon avantageuse, le dispositif selon l'invention permet l'isolement des microorganismes.
L'isolement de microorganismes peut être réalisé à l'aide d'un moyen mécanique en contact avec le dispositif, tel qu'une oëse, ce qui nécessite un glissement continue de l'oëse sur la surface du dispositif à savoir la couche poreuse. Dans ce cas, pour éviter que le moyen d'étalement ne détache la couche poreuse, il est nécessaire d'utiliser un liant et un réticulant dans la couche. Celui-ci réticule les liants et évite que la couche se délite lorsqu'elle est humide. Ainsi, selon un mode particulier de l'invention, la couche poreuse comprend un réticulant. Parmi les réticulants qui peuvent être utilisés on citera : l'iso cyanate, le carbo di imide acétaldéhyde, les sels multivalents, les sels de zirconium, les époxy, les époxy bi fonctionnels, l'anhydride maléique, le copolymère vinyle formamide, le copolymère à base d'éthane diol, les tanins synthétiques, les dispersions aqueuses de polyuréthane, la cyclodextrine, l'oxychlorure de phosphore, le trimetaphosphate de sodium, les glyoxal modifiés, le polyamidoamine epichlorohydrine et la mélamine-Formaldéhyde. Préférentiellement le réticulant est choisi parmi les réticulants suivants : l'iso cyanate et la mélamine-formaldéhyde.
Les inventeurs ont montré que des glissements particulièrement efficaces étaient obtenus lorsque la couche poreuse comprend du kaolin ou du Talc.
Ainsi, le frottement de l'oëse est plus faible sur une surface réalisée avec une couche de kaolin qu'avec les autres pigments. De ce fait l'isolement est facilité. La surface lisse de la couche de kaolin, dont les pigments ont la forme de plaquette, est adaptée à l'isolement réalisé à l'aide d'un moyen d'isolement en contact avec le dispositif. De plus, le kaolin est un aluminosilicate plutôt neutre pour les applications biologiques. Dans un mode de réalisation particulier, le kaolin est alors associé au styrène butadiène comme liant et à l'iso cyanate comme réticulant.

Suivant un mode de réalisation préféré, la couche poreuse est opaque afin de ne pas laisser passer la lumière issue du matériau fibreux. En effet, cela aurait pour inconvénient de perturber la vision des colonies. Avantageusement, le dispositif comprend une première couche poreuse, en contact avec le substrat fibreux, comprenant du dioxyde de titane et une seconde couche poreuse externe comprenant du kaolin. La couche de dioxyde de titane empêche ainsi à la lumière issue du substrat fibreux de passer et permet une meilleure visualisation des colonies, ceci sans empêcher la croissance des colonies.
De la même manière, afin d'améliorer le contraste des colonies, la couleur de la membrane poreuse sera adaptée suivant le moyen de visualisation et l'éclairage. Par exemple, la couche poreuse sera blanche avec une Blancheur CIE supérieure à 65 pour une visualisation à l'oeil nu avec un éclairage frontal. L'ensemble substrat fibreux et couche poreuse blanche a un meilleur contraste et ainsi une meilleur identification du microorganisme cible qu'une gélose.

En outre, le dispositif selon l'invention présente plusieurs avantages écologiques :
- il ne nécessite pas d'agar agar qui est une ressource limitée ;
- il peut être détruit par combustion avec très peu d'énergie et sans dégagement de matière toxique. Sa destruction est donc facile et peu coûteuse, elle peut se faire à proximité du lieu de l'analyse ce qui est intéressant pour un matériel souillé.
- l'ensemble substrat fibreux et couche poreuse pigmentaire peut être réalisé à plus de 95%, avec des matériaux renouvelables.

L'invention concerne également un procédé de fabrication d'un support comprenant une étape de dépôt par couchage sur une des faces d'un substrat fibreux, d'une couche poreuse comprenant un pigment ou un mélange de pigments, et au moins un liant, ledit pigment ayant une taille inférieure à 5 µm et la quantité dudit pigment ou du mélange de pigments étant comprise entre 50 et 97% en poids sec par rapport au poids sec de la couche poreuse.
La couche poreuse peut être déposée par des techniques couchages, à savoir de transfert, de lamination, de couchage sec ou de couchage humide. Parmi les techniques de couchage humide, il existe notamment le couchage au rideau, au ménisque, à la fente, par gravure hélio, par gravure inverse, à l'aide d'une presse encolleuse, au moyen d'une barre de Mayer, par lame, par lame d'air. La couche humide est ensuite séchée par air ou par des techniques radiatives telles que des rayons infra rouges ou des micros ondes. Ainsi la couche et le substrat fibreux sont solidaires. Ils forment un tout et ne peuvent pas être séparés sans qu'il y ait une détérioration du substrat et/ou de la couche.

Une étape de calandrage peut également être effectuée avant et/ou après le couchage.

L'invention porte aussi sur le produit obtenu par ce procédé. Ce produit correspond à un procédé de fabrication d'un produit intermédiaire dans lequel le substrat fibreux ne comprend pas de milieu nutritif.

L'invention concerne également un support destiné à la croissance de microorganisme comprenant
- un substrat fibreux hydrophile;
- au moins une couche poreuse déposée sur l'une des faces du substrat fibreux, comprenant un pigment ou un mélange de pigments, et au moins un liant, ledit pigment ayant une taille inférieure à 5 µm et la quantité dudit pigment ou du mélange de pigments étant comprise entre 50 et 97% en poids sec par rapport au poids sec de la couche poreuse ;

Préférentiellement, le substrat fibreux comprend des fibres de cellulose, notamment de coton.
Encore préférentiellement, les pigments sont des pigments inorganiques choisis parmi les pigments suivants : kaolin, talc, dioxyde de titane, carbonate de calcium. Avantageusement, la quantité dudit pigment ou du mélange de pigments est comprise entre 50 et 97% en poids sec par rapport au poids sec de la couche poreuse, préférentiellement entre 60 et 95%, encore préférentiellement entre 80 et 90%.
De préférence, le liant est du latex styrène butadiène et/ou du latex styrène acrylique et/ou du carboxyle méthyle cellulose, et le réticulant est choisi parmi les réticulants suivants : l'iso cyanate et la mélamine-formaldéhyde.
Dans un mode de réalisation particulier, le kaolin est associé au styrène butadiène comme liant et à l'isocyanate comme réticulant.
Dans un autre mode de réalisation particulier, le dispositif comprend une première couche poreuse, en contact avec le substrat fibreux, comprenant du dioxyde de titane et une seconde couche poreuse externe comprenant du kaolin.

L'invention concerne également un procédé de fabrication d'un dispositif selon l'invention comprenant une étape préalable ou ultérieure à l'étape de couchage, de mise en contact du substrat fibreux hydrophile avec un milieu nutritif.
L'invention porte aussi sur le produit obtenu par ce procédé.
Le substrat fibreux peut être imprégné par un milieu nutritif déshydraté ou liquide.
Lorsque, avant d'être imprégné par le milieu nutritif, le substrat fibreux est déjà recouvert d'une couche poreuse, l'imprégnation se fait par la surface du substrat fibreux exempte de couche poreuse. L'imprégnation est alors facilitée et ne détériore pas les propriétés de la couche poreuse.

L'imprégnation par un milieu déshydraté peut se faire selon la méthode décrite dans la demande de brevet FR 1450149. Lorsque le substrat fibreux est imprégné d'un milieu liquide ou semi liquide, il peut être séché pour fournir un dispositif selon l'invention avec un milieu déshydraté.

L'invention concerne également l'utilisation d'un dispositif selon l'invention pour cultiver et/ou isoler et/ou détecter et/ou identifier et/ou dénombrer au moins un micro-organisme cible, de préférence au moins une bactérie cible, dans un échantillon susceptible de le contenir.
De façon avantageuse, le dispositif est utilisé pour cultiver et isoler au moins un micro-organisme cible, de préférence au moins une bactérie cible, dans un échantillon susceptible de le contenir. Préférentiellement, il comprend un réticulant dans la couche poreuse, préférentiellement choisi parmi l'iso cyanate et la mélamine-formaldéhyde. Avantageusement, le kaolin est associé au styrène butadiène comme liant et à l'isocyanate comme réticulant. Dans un autre mode de réalisation particulier, le dispositif comprend une première couche poreuse, en contact avec le substrat fibreux, comprenant du dioxyde de titane et une seconde couche poreuse externe comprenant du kaolin.

L'invention propose un procédé de culture et/ou d'isolement et/ou de détection et/ou d'identification et/ou de dénombrement d'au moins un micro-organisme cible, dans un échantillon susceptible de le contenir, ledit procédé comprenant les étapes suivantes :
a) fournir un dispositif selon l'une quelconque des revendications 1 à 10,
b) déposer un volume déterminé de l'échantillon sur la couche poreuse,
c) incuber le dispositif pendant un temps et à une température prédéterminés permettant la croissance et l'apparition de colonies d'au moins un micro-organisme cible,
d) détecter et/ou identifier et/ou dénombrer les colonies formées;
ledit procédé comprenant également au moins une étape de réhydratation du milieu de culture avec un volume de liquide prédéterminé avant ou simultanément à l'étape b) et/ou c).

La réhydratation se fait par la surface du substrat fibreux exempte de couche poreuse.
Avantageusement, le procédé est caractérisé en ce qu'un dispositif comprenant un réticulant est fourni et qu'après le dépôt de l'échantillon, les micro-organismes sont isolés à l'aide d'un moyen d'isolement. Les microorganismes sont isolés par épuisement ou par nappage de l'échantillon.
Ainsi, le procédé selon l'invention présente l'avantage de permettre l'isolement de microorganismes à l'aide d'un moyen d'isolement tel qu'il est possible de le faire sur un milieu gélosé.

On entend par échantillon une petite partie ou petite quantité séparée d'une entité par un acte soustractif dénommé habituellement prélèvement, à des fins d'analyse. L'échantillon peut être d'origine biologique, humaine, animale, végétale ou environnementale. Il peut concerner un produit au cours d'un process industriel ou un produit fini, par exemple alimentaire. Il peut donc correspondre à un prélèvement de fluide biologique (sang total, sérum, plasma, urine, liquide céphalo-rachidien, sécrétion organique), un prélèvement tissulaire ou des cellules isolées. Il peut être d'origine industrielle, soit, selon une liste non exhaustive un prélèvement d'air, un prélèvement d'eau, un prélèvement effectué sur une surface, une pièce ou un produit en cours de traitement ou manufacturé, un produit d'origine alimentaire. Parmi les échantillons d'origine alimentaire, on peut citer de façon non exhaustive un échantillon de produits lactés (yaourts, fromages...), de viande, de poisson, d'oeufs, de fruits, de légumes, d'eau, de boisson (lait, jus de fruits, soda, etc) et les produits constitutifs ou annexes du produit fini. Un échantillon alimentaire peut enfin être issu d'une alimentation destinée aux animaux, telle que notamment des farines animales. Ce prélèvement peut subir préalablement à son analyse une préparation de type enrichissement, extraction, concentration, purification, selon des méthodes connues de l'homme du métier.
Selon un mode de réalisation préférentiel, le volume d'échantillon déposé sur le milieu de culture est compris entre 10 et 1000µl.

Au sens de la présente invention, le terme microorganisme recouvre les bactéries gram positif ou gram négatif, les levures, les moisissures, les amibes et plus généralement, les organismes unicellulaires, invisibles à l'oeil nu, qui peuvent être manipulés et multipliés en laboratoire.
Selon un mode préféré de réalisation de l'invention, le microorganisme est une bactérie, gram négative ou positive, ou une levure.
A titre de bactéries Gram positives, on peut citer les bactéries des genres suivants : *Enterococcus, Streptococcus, Lactobacillus, Bifidobacterium, Staphylococcus, Bacillus, Listeria*, *Clostridium, Mycobacteria, Nocardia, Corynebacteria, Micrococcus et Deinococcus.*
A titre de levures, on peut citer les levures des genres suivants : *Candida, Cryptococcus, Saccharomyces et Trichosporon.*
A titre de moisissures, on peut citer les moisissures des genres suivants : *Aspergillus, Penicillium , Cladosporium.*
A titre de bactéries Gram négatives on peut citer les bactéries des genres suivants : *Salmonella, Escherichia coli et Pseudomonas.*

Par milieu nutritif, on entend un milieu comprenant tous les éléments nécessaires à la survie et/ou à la croissance des micro-organismes. Le milieu selon l'invention peut comprendre, par exemple : des peptones, un ou plusieurs facteurs de croissance, des hydrates de carbone, un ou plusieurs agents sélectifs, des tampons, un ou plusieurs gélifiants, etc. De manière générale, le milieu peut en sus contenir un substrat permettant la détection d'une activité enzymatique ou métabolique des micro-organismes cibles grâce à un signal détectable directement ou indirectement. Ce substrat peut être lié à une partie faisant office de marqueur, fluorescent ou chromogène. A titre d'exemple de chromophore, l'on peut citer le rouge neutre, le bleu d'aniline, le bleu de bromocresol.
La détection des bactéries permet de déceler, à l'oeil nu ou à l'aide d'un appareil optique, l'existence d'une croissance des bactéries cibles, à savoir l'apparition de colonies colorées et/ou fluorescentes (selon que l'on utilise un substrat chromogène, fluorogène ou présentant les deux caractéristiques à la fois).

Tel qu'indiqué précédemment, la détection de la fluorescence émise après clivage des substrats enzymatiques fluorogènes impose le recours à un appareil optique, alors que la visualisation du clivage des substrats enzymatiques chromogènes peut être effectuée à l'oeil nu ou, au besoin, à l'aide d'un appareil optique.
Avantageusement, la détection des bactéries permet également leur identification et/ou leur dénombrement.
Préférentiellement, l'identification se fait par analyse spectrale.

Le dénombrement des bactéries consiste, quant à lui, à quantifier le nombre de colonies de bactéries ayant poussé sur le milieu de culture en mettant en oeuvre des techniques de microbiologie bien connues de l'homme du métier.

L'invention, sa fonctionnalité, ses applications ainsi que ses avantages seront mieux appréhendés à la lecture de la description suivante comportant plusieurs exemples comparatifs et dans lesquelles :
- la couche T est une solution aqueuse composée de 1 part de carboxyle méthyle cellulose, 5 parts de latex styrène butadiène, 0,5 part de dispersant polymérique acrylate pour 100 parts de pigments de dioxyde de titane (Tiona AT1).
- La couche PU est une solution aqueuse composée d'une part de carboxyle méthyle cellulose, 12 parts de latex styrène butadiène, 100 parts de billes de polyuréthane (Decosoft 7D Microchem, Erlenbach Switzerland), 1 part de dispersant polymérique acrylate, 1 part d'antimousse, 0,9 part de réticulant iso cyanate.
- La couche K1 est une solution aqueuse composée d'une part de carboxyle méthyle cellulose, 12 parts de latex styrène butadiène, 100 parts de pigment de Kaolin (Capim RG Imerys Rio Capim Caulim), 1 part d'antimousse, 0,9 part de réticulant iso cyanate.
- La couche K2 est une solution aqueuse composée d'une part de carboxyle méthyle cellulose, 20 parts de latex styrène butadiène, 100 parts de pigment de Kaolin (Capim RG Imerys Rio Capim Caulim), 1,5 part de réticulant iso cyanate.

### Exemple 1

Substrat fibreux non recouvert d'une couche poreuse.

Un substrat fibreux fabriqué par la société Arjowiggins Creative Papers de 400g/m² constitué de fibres de coton fabriquées sur une machine à papier est stérilisé, placé dans une boite de Pétri et imprégné de nutriments (milieu Chrom ID^{™} CPS 3) (bioMéreux Ref 43541) sur la face du substrat fibreux exempte de la couche poreuse. Un inoculum contenant la bactérie Escherichia coli est appliqué à l'oëse suivant la méthode des cadrans. L'ensemble est placé dans une étuve à 37°C pendant 24h.

On observe une croissance bactérienne au sein du matériau fibreux. Il n'est pas possible d'identifier ou de compter des colonies.

### Exemple 2

Substrat fibreux recouvert d'une couche constituée de billes de polyuréthane de taille comprise entre 6 et 9 micronmètres.

Un substrat fibreux fabriqué par la société Arjowiggins Creative Papers de 400g/m² constitué de fibres de coton fabriquées sur une machine à papier est couché à la barre de Mayer avec 30g/m² sec de couche T puis 20g/m² sec de couche PU. L'ensemble est stérilisé, placé dans une boite de Pétri et imprégné de nutriments (milieu Chrom ID CPS 3) (bioMéreux Ref 43541) sur la face du substrat fibreux exempte de la couche poreuse.

Sur un échantillon, un inoculum contenant la bactérie Escherichia coli est appliqué à l'oëse suivant la méthode des cadrans, sur un autre échantillon un inoculum contenant la bactérie Escherichia coli est dilué et étalé au râteau sur la surface du produit.

L'inoculum est déposé sur la couche poreuse.

Les échantillons sont placés dans une étuve à 37°C pendant 24h.

Les colonies se développent en profondeur et ne sont pas bien identifiables sur l'échantillon d'isolement.

Il n'y a pas de colonies isolées sur l'échantillon d'énumération.

### Exemple 3

Substrat fibreux avec une couche en contact avec le substrat contenant du dioxyde de Titane et une couche de surface contenant du Kaolin.

Un substrat fibreux fabriqué par la société Arjowiggins Creative Papers de 400g/m² constitué de fibres de coton fabriquées sur une machine à papier est couché à la barre de Mayer avec 30g/m² sec de couche T puis 20g/m² sec de couche K1. L'ensemble est stérilisé, placé dans une boite de Pétri et imprégné de nutriments (milieu Chrom ID CPS 3) (bioMéreux Ref 43541) sur la face du substrat fibreux exempte de la couche poreuse.
L'inoculum est déposé sur la couche poreuse :
Sur un échantillon 3A un inoculum contenant la bactérie Enterococcus faecalis est dilué et étalé au râteau sur la surface du produit.
Sur un échantillon 3B un inoculum contenant la bactérie Citrobacter freundii est dilué et étalé au râteau sur la surface du produit.
Sur un échantillon 3C un inoculum contenant la bactérie Serratia marcescens est dilué et étalé au râteau sur la surface du produit.
Sur un échantillon 3D, un inoculum contenant la bactérie Escherichia coli est appliqué à l'oëse suivant la méthode des cadrans.
Sur un échantillon 3E, un inoculum contenant la bactérie Staphylococcus aureus est appliqué à l'oëse suivant la méthode des cadrans.
Sur un échantillon 3F, un inoculum contenant la bactérie Klebsiella pneumoniae est appliqué à l'oëse suivant la méthode des cadrans.
Sur un échantillon 3G, un inoculum contenant la bactérie Pseudomonas aeruginosa est appliqué à l'oëse suivant la méthode des cadrans.
Sur un échantillon 3H, un inoculum contenant la bactérie Enterobacter cloacae est appliqué à l'oëse suivant la méthode des cadrans.
Sur un échantillon 3I, un inoculum contenant la bactérie Acinetobacter baumanii est appliqué à l'oëse suivant la méthode des cadrans.

Les échantillons sont placés dans une étuve à 37°C pendant 24h.
La blancheur CIE humide du produit est 68.
Les colonies des échantillons d'énumération 3A, 3B et 3C sont bien rondes et isolées et peuvent être comptées.
Les colonies sur les échantillons d'isolement 3D, 3E, 3F, 3G, 3H et 3I sont bien rondes et isolées, le morphotype est respecté.

### Exemple 4

Substrat fibreux avec une couche du bas contenant du dioxyde de Titane et une couche du haut contenant du Kaolin appliquée par 3 couchages successifs.

Un substrat fibreux fabriqué par la société Arjowiggins Creative Papers de 400g/m² constitué de fibres de coton fabriquées sur une machine à papier est couché à la barre de Mayer avec 30g/m² sec de couche T puis trois fois 20g/m² sec de couche K1. L'ensemble est stérilisé, placé dans une boite de Pétri et imprégné de nutriments (milieu Chrom ID CPS 3) (bioMéreux Ref 43541) sur la face du substrat fibreux exempte de la couche poreuse.

L'inoculum est déposé sur la couche poreuse :

Un inoculum contenant la bactérie Escherichia coli est appliqué à l'oëse suivant la méthode des cadrans. L'échantillon est placé dans une étuve à 37°C pendant 24h.

Les colonies sont bien rondes et isolées.

### Exemple 5

Substrat fibreux couché et imprégné d'un polymère hydrosoluble afin d'épaissir le nutriment pour réduire les mouvements latéraux du liquide au sein du substrat.

Un substrat fibreux fabriqué par la société Arjowiggins Creative Papers de 400g/m² constitué de fibres de coton fabriquées sur une machine à papier est imprégné de 30g/m² sec d'hydroxyethyl cellulose (cellosize WP 09L) couché à la barre de Mayer avec 30g/m² sec de couche T puis 20g/m² sec de couche K1. L'ensemble est stérilisé, placé dans une boite de Pétri et imprégné de nutriments (milieu Chrom ID CPS 3) (bioMéreux Ref 43541) sur la face du substrat fibreux exempte de la couche poreuse.

L'inoculum est déposé sur la couche poreuse :

Sur un échantillon 5A, un inoculum contenant la bactérie Escherichia coli est appliqué à l'oëse suivant la méthode des cadrans.

Sur un échantillon 5B un inoculum contenant la bactérie Escherichia coli dilué et étalé au râteau sur la surface du produit.

Sur un échantillon 5C un inoculum contenant la bactérie Enterococcus faecalis dilué et étalé au râteau sur la surface du produit.

Sur un échantillon 5D un inoculum contenant la bactérie Serratia marcescens dilué et étalé au râteau sur la surface du produit.

Sur un échantillon 5E un inoculum contenant la bactérie Citrobacter freundii dilué et étalé au râteau sur la surface du produit.

Les échantillons sont placés dans une étuve à 37°C pendant 24h.

Les colonies sur l'échantillon d'isolement 5A sont bien rondes et isolées.

Les colonies des échantillons d'énumération 5B, 5C, 5D et 5E sont bien rondes et isolées et peuvent être comptées, le morphotype est respecté.

### Exemple 6

Substrat fibreux recouvert d'une seule couche constituée de kaolin.

Un substrat fibreux fabriqué par la société Arjowiggins Creative Papers de 400g/m² constitué de fibres de coton fabriquées sur une machine à papier est couché à la barre de Mayer avec 26g/m² sec de couche K2. L'ensemble est stérilisé, placé dans une boite de Pétri et imprégné de nutriments (milieu Chrom ID CPS 3) (bioMéreux Ref 43541) sur la face du substrat fibreux exempte de la couche poreuse.

L'inoculum est déposé sur la couche poreuse :

Sur un échantillon 6A, un inoculum contenant la bactérie Escherichia coli est appliqué à l'oëse suivant la méthode des cadrans.

Sur un échantillon 6B un inoculum contenant la bactérie Escherichia coli dilué et étalé au râteau sur la surface du produit.

Les échantillons sont placés dans une étuve à 37°C pendant 24h.

Les colonies sur l'échantillon d'isolement 6A sont rondes et isolées.

Les colonies des échantillons d'énumération 6B sont rondes et isolées et peuvent être comptées.

## Revendications

1. Dispositif pour la culture et/ou l'isolement et/ou la détection et/ou l'identification et/ou le dénombrement d'au moins un microorganisme cible dans un échantillon susceptible de le contenir **caractérisé en ce qu'**il comprend un support et un milieu nutritif ;
ledit support comprenant :
• un substrat fibreux hydrophile,
• au moins une couche poreuse en contact avec l'une des faces du substrat fibreux, comprenant un pigment ou un mélange de pigments, et au moins un liant, ledit pigment ayant une taille inférieure à 5 µm et la quantité dudit pigment ou dudit mélange de pigments étant comprise entre 50 et 97% en poids sec par rapport au poids sec de la couche poreuse ;
ledit milieu nutritif étant compris dans le substrat fibreux.

2. Dispositif selon la revendication 1 **caractérisé en ce que** la couche poreuse est solidaire avec l'une des faces du substrat fibreux.

3. Dispositif selon l'une quelconque des revendications 1 ou 2 **caractérisé en ce que** les pigments sont des pigments inorganiques choisis parmi les pigments suivants : kaolin, talc, dioxyde de titane, carbonate de calcium.

4. Dispositif selon l'une quelconque des revendications 1 à 3 **caractérisé en ce que** le liant est choisi parmi les liants suivants : latex styrène butadiène, latex styrène acrylique, carboxyle méthyle cellulose.

5. Dispositif selon l'une quelconque des revendications 1 à 4 **caractérisé en ce que** la quantité de liant est comprise entre 3 et 25% en poids sec par rapport au poids sec de la couche poreuse.

6. Dispositif selon l'une quelconque des revendications 1 à 5 comprenant
• une première couche poreuse, en contact avec le substrat fibreux, comprenant du dioxyde de titane et
• une seconde couche poreuse externe comprenant du kaolin.

7. Dispositif selon l'une quelconque des revendications 1 à 6 **caractérisé en ce que** le substrat fibreux comprend des fibres de cellulose, préférentiellement de coton.

8. Dispositif selon l'une quelconque des revendications 1 à 7 **caractérisé en ce que** le milieu nutritif est un milieu déshydraté.

9. Dispositif selon l'une quelconque des revendications 1 à 8 **caractérisé en ce que** la couche poreuse comprend un réticulant.

10. Dispositif selon la revendication 9 **caractérisé en ce que** le réticulant est choisi parmi les réticulants suivants : l'iso cyanate et la mélamine-formaldéhyde.

11. Procédé de fabrication d'un support comprenant une étape de dépôt par couchage sur une des faces d'un substrat fibreux hydrophile, d'une couche poreuse comprenant un pigment ou un mélange de pigments, et au moins un liant, ledit pigment ayant une taille inférieure à 5 µm et la quantité dudit pigment ou du mélange de pigments étant comprise entre 50 et 97% en poids sec par rapport au poids sec de la couche poreuse.

12. Procédé de fabrication d'un dispositif selon la revendication 11 comprenant une étape préalable ou ultérieure à l'étape de couchage, de mise en contact du substrat fibreux hydrophile avec un milieu nutritif.

13. Produit obtenu par le procédé selon la revendication 11 ou 12.

14. Support destiné à la croissance de microorganisme comprenant
• un substrat fibreux hydrophile;
• au moins une couche poreuse déposée sur l'une des faces du substrat fibreux, comprenant un pigment ou un mélange de pigments, et au moins un liant, ledit pigment ayant une taille inférieure à 5 µm et la quantité dudit pigment ou du mélange de pigments étant comprise entre 50 et 97% en poids sec par rapport au poids sec de la couche poreuse ;

15. Utilisation d'un dispositif selon l'une quelconque des revendications 1 à 10 pour cultiver et/ou isoler et/ou détecter et/ou identifier et/ou dénombrer au moins un micro-organisme cible, de préférence au moins une bactérie cible, dans un échantillon susceptible de le contenir.

16. Procédé de culture et/ou d'isolement et/ou de détection et/ou d'identification et/ou de dénombrement d'au moins un micro-organisme cible, dans un échantillon susceptible de le contenir, ledit procédé comprenant les étapes suivantes :
e) fournir un dispositif selon l'une quelconque des revendications 1 à 10,
f) déposer un volume déterminé de l'échantillon sur la couche poreuse,
g) incuber le dispositif pendant un temps et à une température prédéterminés permettant la croissance et l'apparition de colonies d'au moins un micro-organisme cible,
h) détecter et/ou identifier et/ou dénombrer les colonies formées;
ledit procédé comprenant également au moins une étape de réhydratation du milieu de culture avec un volume de liquide prédéterminé avant ou simultanément à l'étape b) et/ou c).

17. Procédé selon la revendication 16 **caractérisé en ce qu'**un dispositif selon la revendication 9 est fourni et qu'après le dépôt de l'échantillon, les micro-organismes sont isolés à l'aide d'un moyen d'isolement.

18. Procédé selon l'une quelconque des revendications 16 ou 17 **caractérisé en ce que** l'identification se fait par analyse spectrale.

## Patentansprüche

1. Vorrichtung für die Kultur und/oder die Isolierung und/oder den Nachweis und/oder die Identifikation und/oder die zahlenmäßige Bestimmung von mindestens einem Zielmikroorganismus in einer Probe, die diesen enthalten kann, **dadurch gekennzeichnet, dass** sie einen Träger und ein Nährmedium umfasst;
wobei der Träger Folgendes umfasst:
• ein faserartiges hydrophiles Substrat,
• mindestens eine poröse Schicht, die mit einer der Flächen des faserartigen Substrats in Kontakt steht, umfassend ein Pigment oder eine Mischung von Pigmenten sowie mindestens ein Bindemittel, wobei das Pigment eine Größe von unter 5 µm aufweist und wobei die Menge des Pigments oder der Mischung von Pigmenten zwischen 50 und 97 Trockengewichts-% in Bezug auf das Trockengewicht der porösen Schicht beträgt;
wobei das Nährmedium in dem faserartigen Substrat vorliegt.

2. Vorrichtung nach Anspruch 1, **dadurch gekennzeichnet, dass** die poröse Schicht mit einer der Flächen des faserartigen Substrats verbunden ist.

3. Vorrichtung nach einem der Ansprüche 1 oder 2, **dadurch gekennzeichnet, dass** es sich bei den Pigmenten um anorganische Pigmente, ausgewählt aus den folgenden Pigmenten, handelt: Kaolin, Talk, Titandioxid, Calciumcarbonat.

4. Vorrichtung nach einem der Ansprüche 1 bis 3, **dadurch gekennzeichnet, dass** das Bindemittel aus den folgenden Bindemitteln ausgewählt ist: Styrol/Butadien-Latex, Styrol/Acrylsäurelatex, Carboxymethylcellulose.

5. Vorrichtung nach einem der Ansprüche 1 bis 4, **dadurch gekennzeichnet, dass** die Menge an Bindemittel zwischen 3 und 25 Trockengewichts-% in Bezug auf das Trockengewicht der porösen Schicht beträgt.

6. Vorrichtung nach einem der Ansprüche 1 bis 5, umfassend
• eine erste poröse Schicht, die in Kontakt mit dem faserartigen Substrat steht und die Titandioxid umfasst, und
• eine zweite äußere poröse Schicht, die Kaolin umfasst.

7. Vorrichtung nach einem der Ansprüche 1 bis 6, **dadurch gekennzeichnet, dass** das faserartige Substrat Cellulosefasern, vorzugsweise Baumwollfasern, umfasst.

8. Vorrichtung nach einem der Ansprüche 1 bis 7, **dadurch gekennzeichnet, dass** es sich bei dem Nährmedium um ein dehydriertes Medium handelt.

9. Vorrichtung nach einem der Ansprüche 1 bis 8, **dadurch gekennzeichnet, dass** die poröse Schicht ein Vernetzungsmittel umfasst.

10. Vorrichtung nach Anspruch 9, **dadurch gekennzeichnet, dass** das Vernetzungsmittel aus den folgenden Vernetzungsmitteln ausgewählt ist: Isocyanat und Melamin-Formaldehyd.

11. Verfahren zur Herstellung eines Trägers, umfassend einen Auftragungsschritt durch Auftragen einer porösen Schicht umfassend ein Pigment oder eine Mischung von Pigmenten sowie mindestens ein Bindemittel auf einer der Flächen eines faserartigen hydrophilen Substrats, wobei das Pigment eine Größe von unter 5 µm aufweist und wobei die Menge des Pigments oder der Mischung von Pigmenten zwischen 50 und 97 Trockengewichts-% in Bezug auf das Trockengewicht der porösen Schicht beträgt.

12. Verfahren zur Herstellung einer Vorrichtung nach Anspruch 11, umfassend einen Schritt vor oder nach dem Auftragungsschritt, in dem man das faserartige hydrophile Substrat mit einem Nährmedium in Kontakt bringt.

13. Produkt, erhalten nach dem Verfahren nach Anspruch 11 oder 12.

14. Träger für das Wachstum von Mikroorganismen, umfassend:
• ein faserartiges hydrophiles Substrat,
• mindestens eine poröse Schicht, die auf einer der Flächen des faserartigen Substrats aufgetragen ist, umfassend ein Pigment oder eine Mischung von Pigmenten sowie mindestens ein Bindemittel, wobei das Pigment eine Größe von unter 5 µm aufweist und wobei die Menge des Pigments oder der Mischung von Pigmenten zwischen 50 und 97 Trockengewichts-% in Bezug auf das Trockengewicht der porösen Schicht beträgt.

15. Verwendung einer Vorrichtung nach einem der Ansprüche 1 bis 10 zum Kultivieren und/oder Isolieren und/oder Nachweisen und/oder Identifizieren und/oder zahlenmäßige Bestimmen von mindestens einem Zielmikroorganismus, vorzugsweise von mindestens einem Zielbakterium, in einer Probe, die diesen enthalten kann.

16. Verfahren für die Kultur und/oder die Isolierung und/oder den Nachweis und/oder die Identifikation und/oder die zahlenmäßige Bestimmung von mindestens einem Zielmikroorganismus in einer Probe, die diesen enthalten kann, wobei das Verfahren die folgenden Schritte umfasst:
e) Bereitstellen einer Vorrichtung nach einem der Ansprüche 1 bis 10,
f) Auftragen eines bestimmten Volumens der Probe auf die poröse Schicht,
g) Inkubieren der Vorrichtung über einen vorbestimmten Zeitraum und bei einer vorbestimmten Temperatur, die das Wachstum und das Auftreten von Kolonien von mindestens einem Zielmikroorganismus gestatten,
h) Nachweisen und/oder Identifizieren und/oder zahlenmäßiges Bestimmen der gebildeten Kolonien;
wobei das Verfahren auch mindestens einen Schritt der Rehydratation des Kulturmediums mit einem vorbestimmten Flüssigkeitsvolumen vor oder gleichzeitig mit Stufe b) und/oder c) umfasst.

17. Verfahren nach Anspruch 16, **dadurch gekennzeichnet, dass** eine Vorrichtung nach Anspruch 9 bereitgestellt wird und dass nach dem Auftragen der Probe die Mikroorganismen mit Hilfe eines Mittels zum Isolieren isoliert werden.

18. Verfahren nach einem der Ansprüche 16 oder 17, **dadurch gekennzeichnet, dass** die Identifikation mittels Spektralanalyse erfolgt.

## Claims

1. A device for the culturing and/or isolation and/or detection and/or identification and/or counting of at least one target microorganism in a sample liable to contain it, **characterized in that** it comprises a support and a nutrient medium;
said support comprising:
• a hydrophilic fibrous substrate,
• at least one porous layer in contact with one of the faces of the fibrous substrate, comprising a pigment or a mixture of pigments and at least one binder, said pigment having a size of less than 5 µm and the amount of said pigment or said mixture of pigments being between 50 and 97% by dry weight, with respect to the dry weight of the porous layer;
said nutrient medium being included in the fibrous substrate.

2. The device as claimed in claim 1, **characterized in that** the porous layer is integral with one of the faces of the fibrous substrate.

3. The device as claimed in either one of claims 1 and 2, **characterized in that** the pigments are inorganic pigments chosen from the following pigments: kaolin, talc, titanium dioxide and calcium carbonate.

4. The device as claimed in any one of claims 1 to 3, **characterized in that** the binder is chosen from the following binders: styrene-butadiene latex, styrene-acrylic latex and carboxymethyl cellulose.

5. The device as claimed in any one of claims 1 to 4, **characterized in that** the amount of binder is between 3 and 25% by dry weight, with respect to the dry weight of the porous layer.

6. The device as claimed in any one of claims 1 to 5, comprising:
• a first porous layer, in contact with the fibrous substrate, containing titanium dioxide, and
• a second external porous layer comprising kaolin.

7. The device as claimed in any one of claims 1 to 6, **characterized in that** the fibrous substrate comprises cellulose fibers; preferably cotton fibers.

8. The device as claimed in any one of claims 1 to 7, **characterized in that** the nutrient medium is a dehydrated medium.

9. The device as claimed in any one of claims 1 to 8, **characterized in that** the porous layer comprises a crosslinking agent.

10. The device as claimed in claim 9, **characterized in that** the crosslinking agent is chosen from the following crosslinking agents: isocyanate and melamine-formaldehyde.

11. A process for the manufacture of a support comprising a stage of deposition by coating, on one of the faces of a hydrophilic fibrous substrate, of a porous layer comprising a pigment or a mixture of pigments and at least one binder, said pigment having a size of less than 5 µm and the amount of said pigment or of the mixture of pigments being between 50 and 97% by dry weight, with respect to the dry weight of the porous layer.

12. The process for the manufacture of a device as claimed in claim 11, comprising a stage, prior or subsequent to the coating stage, of bringing the hydrophilic fibrous substrate into contact with a nutrient medium.

13. A product obtained by the process as claimed in claim 11 or 12.

14. A support intended for the growth of microorganisms comprising:
• a hydrophilic fibrous substrate;
• at least one porous layer deposited on one of the faces of the fibrous substrate, comprising a pigment or a mixture of pigments and at least one binder, said pigment having a size of less than 5 µm and the amount of said pigment or of the mixture of pigments being between 50 and 97% by dry weight, with respect to the dry weight of the porous layer.

15. The use of a device as claimed in any one of claims 1 to 10 for culturing and/or isolating and/or detecting and/or identifying and/or counting at least one target microorganism, preferably at least one target bacterium, in a sample liable to contain it.

16. A process for the culturing and/or isolation and/or detection and/or identification and/or counting of at least one target microorganism, in a sample liable to contain it, said process comprising the following stages:
a) providing a device as claimed in any one of claims 1 to 10,
b) depositing a predetermined volume of the sample on the porous layer,
c) incubating the device for a predetermined time and at a predetermined temperature which make possible the growth and the appearance of colonies of at least one target microorganism,
d) detecting and/or identifying and/or counting the colonies formed;
said process also comprising at least one stage of rehydration of the culture medium with a predetermined volume of liquid, before or simultaneously with stage b) and/or c).

17. The process as claimed in claim 16, **characterized in that** a device as claimed in claim 9 is provided and that, after the deposition of the sample, the microorganisms are isolated using an isolation means.

18. The process as claimed in either one of claims 16 or 17, **characterized in that** the identification takes place by spectral analysis.
